(19) **Europäisches Patentamt / European Patent Office / Office européen des brevets**

(11) **EP 4 733 388 A1**

(12) **EUROPEAN PATENT APPLICATION**
published in accordance with Art. 153(4) EPC

(43) Date of publication:
29.04.2026 Bulletin 2026/18

(21) Application number: 24825329.6

(22) Date of filing: 21.06.2024

(51) International Patent Classification (IPC):
*C12N 1/20* (2026.01)    *A23L 33/135* (2016.01)
*A61K 35/747* (2015.01)    *A61P 3/10* (2006.01)
*A61P 39/06* (2006.01)    *A61P 37/02* (2006.01)
*C12R 1/225* (2006.01)    *C12P 7/56* (2006.01)

(52) Cooperative Patent Classification (CPC):
A23L 33/135; A61K 35/747; A61P 3/10;
A61P 37/02; A61P 39/06; C12N 1/20; C12P 7/56;
C12R 2001/225

(86) International application number:
PCT/CN2024/100580

(87) International publication number:
WO 2024/260442 (26.12.2024 Gazette 2024/52)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 21.06.2023 CN 202310744495
21.06.2023 CN 202310745739
21.06.2023 CN 202310744266

(71) Applicant: COREE Company Limited
Hong Kong 999077 (HK)

(72) Inventors:
• ZHANG, Di
Beijing 101300 (CN)
• ZHANG, Shiqi
Beijing 101300 (CN)
• WANG, Tingting
Beijing 101300 (CN)
• GE, Yangyang
Beijing 101300 (CN)
• ZHANG, Jingjing
Beijing 101300 (CN)
• DING, Yan
Beijing 101300 (CN)
• LEE, Soowon
Beijing 101300 (CN)
• XU, Ying
Beijing 101300 (CN)
• LIM, Chongyoon
Beijing 101300 (CN)

(74) Representative: Studio Torta S.p.A.
Via Viotti, 9
10121 Torino (IT)

Remarks:
The complete document including Reference Table(s) and the Sequence Listing(s) can be downloaded from the EPO website

(54) **LACTOBACILLUS RHAMNOSUS STRAIN, VIABLE BACTERIA FORMULATION AND PREPARATION METHOD THEREFOR AND USE THEREOF**

(57) The present invention relates to the technical field of microorganism, in particular to a *Lactobacillus rhamnosus* strain, a viable bacteria preparation, and a preparation method and use thereof. The deposit number of the *L. rhamnosus* strain is CGMCC No. 25682. The *L. rhamnosus* strain and the viable bacteria preparation thereof of the present invention have excellent free radical scavenging, responding to oxidative stress state, antioxidation and anti-aging ability. They also have the potential to regulate blood glucose, modulate immunity, and are used to maintain the balance of intestinal flora.

EP 4 733 388 A1

Isolation and Screening of
Lactobacillus rhamnosus HOM1213

Fermentation and lyophilization
processes of L. rhamnosus
HOM1213: high viable bacteria count

In vitro screening of L. rhamnosus HOM1213:
tolerance to artificial gastrointestinal fluid,
antibacterial activity, immunomodulation,
and antioxidant activity

In vitro hypoglycemic efficacy study of L.
rhamnosus HOM1213: DPP-4, α-glucosidase
inhibition, and GLP-1 secretion

Animal experiments of L. rhamnosus
HOM1213: increasing the antioxidant levels
in mice with oxidative damage,
and delaying aging

FIG. 5

**Description**

**CROSS-REFERENCE TO RELATED APPLICATIONS**

This application claims priority to Chinese Patent Application No. 202310744495.8 filed in China on June 21, 2023, Chinese Patent Application No. 202310745739.4 filed in China on June 21, 2023, and Chinese Patent Application No. 202310744266.6 filed in China on June 21, 2023, the entire contents of which are incorporated herein by reference.

**TECHNICAL FIELD**

The present invention relates to the technical field of microorganisms, in particular to a *Lactobacillus rhamnosus* strain, a viable bacteria preparation, and a preparation method and use thereof.

**BACKGROUND**

The aging problem is serious in the world today, and delaying aging has become a focus of attention. With the development of science and technology, there has been some consensus on the study of the aging mechanism. The most studied is the free radical-oxidative stress theory, which posits that under normal conditions, free radicals (superoxide anion radical ($O^{2-}$), hydroxyl radical (OH), etc.) are in equilibrium with antioxidant systems. Once the body is stimulated, the free radical content increases, or the ability to scavenge free radicals decreases, the antioxidant enzyme level decreases, damaging cellular components of the body, resulting in lipid peroxidation and protein damage, and an oxidative stress state, until apoptosis and aging, etc.

Diabetes, especially type II diabetes, is currently one of the health problems of global concern, manifested as metabolic diseases of hyperglycemia caused by insufficient insulin secretion. Currently, blood glucose levels in diabetic patients can be controlled by drugs, such as α-glucosidase inhibitors and dipeptidyl peptidase-4 inhibitors (i.e., DPP-4 inhibitors).

Studies have shown that oxidative damage of pancreatic β-cells is one of the causes of diabetes. Excessive free radicals will attack insulin-specific receptors on liver cell membranes, thereby causing insulin resistance, and the ability of the body's antioxidant defense system to scavenge free radicals is significantly reduced, resulting in a series of oxidative injuries.

At present, the research on *Lactobacillus rhamnosus* strains in lowering blood glucose, antioxidation, anti-aging and improving immunity is insufficient in the related art.

**SUMMARY**

In order to solve the above technical problems, the present invention provides a *Lactobacillus rhamnosus* (*L. rhamnosus*) strain, a fermentation product thereof, a viable bacteria preparation, and a preparation method and use thereof. The *L. rhamnosus* strain, the fermentation product thereof or the viable bacteria preparation exhibits excellent properties in antioxidation, anti-aging, lowering blood glucose and improving immunity.

In one aspect, the present invention provides a *L. rhamnosus* strain, and the deposit number of the *L. rhamnosus* strain is CGMCC No. 25682.

In one example, the *L. rhamnosus* strain comprises a 16S rRNA gene sequence represented by SEQ ID NO: 1.

In another aspect, the present invention provides a fermentation product obtained by fermentation using the *L. rhamnosus* strain as described above.

In one example, the fermentation product is a fermented bovine milk product; optionally, the content of viable *L. rhamnosus* strain in the fermented bovine milk product is higher than $6.0 \times 10^8$ CFU/mL.

In another example, the fermentation product is a fermented oat milk product; optionally, the count of viable *L. rhamnosus* strain in the fermented oat milk product is higher than $5.0 \times 10^8$ CFU/mL.

In another aspect, the present invention provides a viable bacteria preparation comprising the *L. rhamnosus* strain as described above.

In one example, the viable bacteria preparation comprises $3.0 \times 10^{11}$ CFU/g or more of the viable bacteria.

In yet another aspect, the present invention provides a food or health care product comprising the viable bacteria preparation as described above.

In one example, the food is selected from one or more of a group consisting of a milk powder, solid beverage, fermented dairy product, dairy-containing beverage and cheese.

In one example, the fermented dairy product is selected from a group consisting of fermented bovine milk products and fermented oat milk products.

Optionally, the content of the viable *L. rhamnosus* strain in the fermented bovine milk product is $6.0 \times 10^8$

CFU/mL or more. Optionally, the count of the viable *L. rhamnosus* strain in the fermented oat milk product is $5.0 \times 10^8$ CFU/mL or more.

In yet another aspect, the present invention provides use of the *L. rhamnosus* strain as described above in the preparation of a medicament for scavenging free radicals, responding to oxidative stress state, antioxidation and anti-aging.

In one example, the *L. rhamnosus* strain is used to increase the levels of superoxide dismutase, glutathione peroxidase and glutathione, and reduce the level of malondialdehyde.

In another example, the *L. rhamnosus* strain is used to scavenge excess free radicals, and increase the scavenging rate of hydroxyl radicals and DPPH radicals.

The present invention also provides use of the *L. rhamnosus* strain as described above in the preparation of a medicament for producing DPP-4 and $\alpha$-glucosidase inhibitors and stimulating NCI-H716 cells to produce GLP-1.

In one example, the *L. rhamnosus* strain is used to assist in lowering blood glucose.

In another aspect, the present invention provides use of the *L. rhamnosus* strain in the preparation of a medicament for modulating immunity.

In one example, the *L. rhamnosus* strain is used to increase the level of cytokines TNF-$\alpha$ and/or IL-6.

In yet another aspect, the present invention provides use of the *L. rhamnosus* strain as described above for increasing the content of lactic acid or short-chain fatty acid.

Preferably, the short-chain fatty acid is acetic acid.

Preferably, the *L. rhamnosus* strain is used to maintain the balance of intestinal flora.

In another aspect, the present invention provides a method for preparing the viable bacteria preparation as described above, comprising the following steps:

propagation - culturing the *L. rhamnosus* strain described above in an optimized liquid medium;
collecting bacteria;
adding protective agent for resuspension, freeze-drying under vacuum, and pulverizing to obtain the viable bacteria preparation.

Advantageous Effects

The *Lactobacillus rhamnosus* strain of the present invention, its fermentation product or the viable bacteria preparation has excellent free radical scavenging, response to oxidative stress state, antioxidation and anti-aging ability, can increase the levels of superoxide dismutase, glutathione peroxidase and glutathione, reduce the level of malondialdehyde, and increase the scavenging rate of hydroxyl radicals and DPPH free radicals; can produce DPP-4 and $\alpha$-glucosidase inhibitors, and stimulate NCI-H716 cells to produce GLP-1, which has the potential to regulate blood glucose; can increase the levels of cytokines TNF-$\alpha$ and IL-6, thus regulating immunity; and can increase the content of lactic acid or short-chain fatty acids for maintaining the balance of intestinal flora. The *L. rhamnosus* of the present invention can also be fermented to produce fermentation products such as fermented bovine milk products and fermented oat milk products. The production process of the viable bacteria preparation of the present invention has simple parameters, and is easy to control and short in cycle, which ensures a high survival rate of *L. rhamnosus*. The obtained product can be stored for a long time with stable quality.

In addition, the *L. rhamnosus* of the present invention has excellent in vitro probiotic function. This strain demonstrates excellent performance in terms of artificial gastrointestinal fluid tolerance, bacteriostatic (inhibiting the proliferation of common pathogenic bacteria), and immune regulation.

**BRIEF DESCRIPTION OF THE DRAWINGS**

FIG. 1 shows RAPD cluster analysis diagrams of *L. rhamnosus* HOM1213 strain and some commercial *L. rhamnosus* strains constructed based on the UPGMA method.

FIG. 2 shows the inhibition rate of *L. rhamnosus* HOM1213 strain of the present invention on DPP-4 and $\alpha$-glucosidase.

FIG. 3 shows the in vitro antioxidant capacity of the *L. rhamnosus* HOM1213 strain of the present invention.

FIG. 4 shows the effect of the *L. rhamnosus* HOM1213 strain of the present invention on the in vivo antioxidant and anti-aging indicators of oxidative damage model mice.

FIG. 5 shows a process flow diagram of the present invention.

**Microbiological Collection**

The *Lactobacillus rhamnosus* (*L. rhamnosus*) HOM1213 strain of the present invention was deposited at the China General Microbiological Culture Collection Center (CGMCC) on September 9, 2022, with a deposit address of: Institute of Microbiology, Chinese Academy of Sciences, No. 3, Courtyard 1, Beichen West Road, Chaoyang District, Beijing; and a deposit number of CGMCC No. 25682.

The *L. rhamnosus* HOM1213 strain of the present invention was sent to the Institute of Microbiology, Chinese Academy of Sciences for identification in May 2023.

The detection and identification conclusions are as follows: Under the conditions of this laboratory, according to the comprehensive analysis of experimental data such as cell morphology, physiological and biochemical characteristics, 16S rRNA gene sequence, and *pheS* gene sequence of the test strain, referring to the *"Bergey's Manual of Systematic Bacteriology"* and relevant research paper of International Journal of Systematic and Evolutionary Microbiology, the identification result of the test strain ( strain number: HOM1213) is: *Lacticaseibacillus rhamnosus.* Synonyms: *Lactobacillus rhamnosus.*

The cell morphology of this strain is: rod-shaped; the physiological and biochemical characteristics are: Gram-positive, catalase negative (-), oxidase negative (-); 16S rRNA gene sequence is shown in SEQ ID NO: 1, and *pheS* gene sequence is shown in SEQ ID NO: 9.

**DETAILED DESCRIPTION**

The technical solutions and effects of the present invention will be further described below in conjunction with specific examples, but are not limited thereto. Any amendments or equivalent substitutions to the technical solutions of the present invention, without departing from the spirit and scope of the technical solutions of the present invention, shall fall within the protection scope of the present invention. The experimental methods without specific conditions in the following embodiments are implemented according to conventional methods and conditions in the art.

It should be noted that the experimental methods used in the present disclosure are conventional methods unless otherwise specified.

Unless otherwise specified, the reagents and materials used in the present invention are all prepared by conventional methods or obtained commercially.

As identified above, *Lactobacillus rhamnosus* and *Lacticaseibacillus rhamnosus* are synonyms, so in the present invention, *Lactobacillus rhamnosus* and *Lacticaseibacillus rhamnosus* can be used interchangeably.

The *Lactobacillus rhamnosus* strain HOM1213 used in the present invention is isolated from the feces of healthy infants, and has the following physicochemical properties: the optimal growth temperature is 37°C, the metabolites are abundant, the *L. rhamnosus* strain HOM1213 has the characteristics of acid and bile salt resistance, bacteriostatic properties, immunomodulatory effects, regulation of blood glucose and antioxidant and anti-aging activities.

In order to further understand the present invention, the *L. rhamnosus* and the use thereof provided by the present invention will be described in detail below in conjunction with examples. The protection scope of the present invention is not limited by the following examples.

**Example**

**Example 1. Isolation and Identification of *L. rhamnosus* Strain HOM1213**

(1) Preparation of Medium

MRS Liquid Medium:

Into MRS medium (OXOID, CM1163), 1L of double-distilled water was added, and stirred evenly. Sterilized at 121°C for 20 min, and then set aside for later use.

Modified MRS Solid Medium:

MRS medium (OXOID, CM1163), 0.05g of bromocresol green (Shanghai Sangon Biotech), 1L of double-distilled water were mixed, and stirred evenly. Adjusted pH to 5.5, sterilized at 121°C for 20 min, and then set aside for later use.

(2) Isolation of Strains

1g of healthy infant feces was weighed, fully shaken and mixed with 9 mL of 0.9% normal saline. The sample was subjected to gradient dilution using a tenfold dilution method. Two suitable dilutions were selected, and 100μL of each of the dilutions was coated on a modified MRS solid plate, and anaerobic culture was performed at 37°C for 72 h. Single colonies with smooth surface, milky white or milky yellow color, and yellowing edges were selected, streaked for culture and purification. Colony morphology was observed by microscopy. Single colonies were selected and cultured in MRS liquid medium for pure culture, preserved in glycerol, and numbered as HOM1213.

(3) Identification of Strains

The strains of the selected strains were inoculated into an MRS liquid medium, cultured at 37°C for 24 h. Total bacterial DNA was extracted from the bacterial solution, and 16S rRNA gene amplification was performed. PCR amplification and agarose gel electrophoresis were performed using universal primers 27F and 1492R. The gel was cut and recovered, and sequenced (Shanghai Sangon Biotech). Then, the HOM1213 strain was identified as *L. rhamnosus* by comparison in the NCBI database using the BLAST tool. The sequence of 16S rRNA gene of HOM1213 strain is shown in SEQ ID NO: 1.

The sequence of 16S rRNA gene of HOM1213 strain is as follows:

TGCAGTCGAACGAGTTCTGATTATTGAAAGGTGCTTGCATCTT

GATTTAATTTTGAACGAGTGGCGGACGGGTGAGTAACACGTGGGTAACCTG

CCCTTAAGTGGGGGATAACATTTGGAAACAGATGCTAATACCGCATAAATCC

AAGAACCGCATGGTTCTTGGCTGAAAGATGGCGTAAGCTATCGCTTTTGGA

TGGACCCGCGGCGTATTAGCTAGTTGGTGAGGTAACGGCTCACCAAGGCAA

TGATACGTAGCCGAACTGAGAGGTTGATCGGCCACATTGGGACTGAGACAC

GGCCCAAACTCCTACGGGAGGCAGCAGTAGGGAATCTTCCACAATGGACG

CAAGTCTGATGGAGCAACGCCGCGTGAGTGAAGAAGGCTTTCGGGTCGTA

AAACTCTGTTGTTGGAGAAGAATGGTCGGCAGAGTAACTGTTGTCGGCGT

GACGGTATCCAACCAGAAAGCCACGGCTAACTACGTGCCAGCAGCCGCGG

TAATACGTAGGTGGCAAGCGTTATCCGGATTTATTGGGCGTAAAGCGAGCG

CAGGCGGTTTTTTAAGTCTGATGTGAAAGCCCTCGGCTTAACCGAGGAAGT

GCATCGGAAACTGGGAAACTTGAGTGCAGAAGAGGACAGTGGAACTCCAT

GTGTAGCGGTGAAATGCGTAGATATGGAAGAACACCAGTGGCGAAGGC

GGCTGTCTGGTCTGTAACTGACGCTGAGGCTCGAAAGCATGGGTAGCGAA

CAGGATTAGATACCCTGGTAGTCCATGCCGTAAACGATGAATGCTAGGTGTT

GGAGGGTTTCCGCCCTTCAGTGCCGCAGCTAACGCATTAAGCATTCCGCCT

GGGGAGTACGACCGCAAGGTTGAAACTCAAAGGAATTGACGGGGGCCCG

CACAAGCGGTGGAGCATGTGGTTTAATTCGAAGCAACGCGAAGAACCTTA

CCAGGTCTTGACATCTTTTGATCACCTGAGAGATCAGGTTTCCCCTTCGGG

GGCAAAATGACAGGTGGTGCATGGTTGTCGTCAGCTCGTGTCGTGAGATGT

TGGGTTAAGTCCCGCAACGAGCGCAACCCTTATGACTAGTTGCCAGCATTT

AGTTGGGCACTCTAGTAAGACTGCCGGTGACAAACCGGAGGAAGGTGGGG

ATGACGTCAAATCATCATGCCCCTTATGACCTGGGCTACACACGTGCTACAA

TGGATGGTACAACGAGTTGCGAGACCGCGAGGTCAAGCTAATCTCTTAAAG

CCATTCTCAGTTCGGACTGTAGGCTGCAACTCGCCTACACGAAGTCGGAAT

CGCTAGTAATCGCGGATCAGCACGCCGCGGTGAATACGTTCCCGGGCCTTG

TACACACCGCCCGTCACACCATGAGAGTTTGTAACACCCGAAGCCGGTGG

CGTAACCCTTTTAGGGAGCGAGCCGTCT

(4) Identification of the Strain by Randomly Amplified Polymorphic DNA(RAPD)

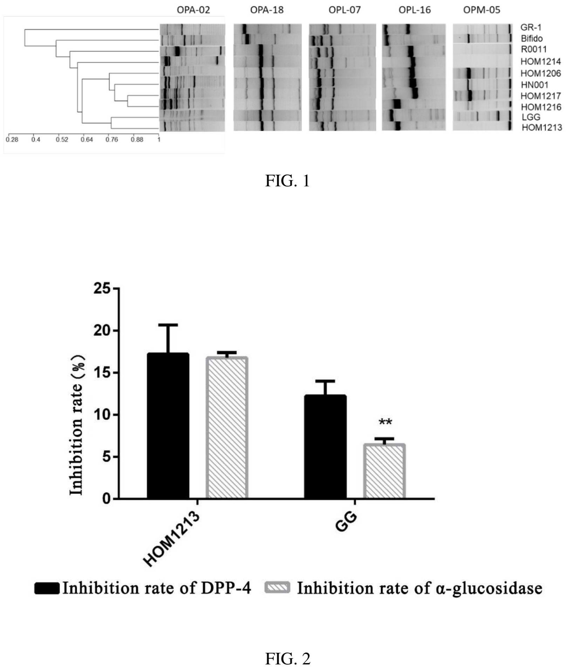

The random polymorphic DNA (RAPD) analysis was performed on the isolated *L. rhamnosus* HOM1213. The results showed that HOM1213 was different from some commercial *L. rhamnosus* strains and exhibited specificity.
DNA was extracted from the preserved strain. Using the strain DNA as a template, 5 primers OPA- 02: TGC CGA GCT G; OPA-18: AGG TGA CCG T; OPL-07: AGG CGG GAA C; OPL-16: AGG TTG CAG G; OPM-05: GGG AAC GTG T were used for PCR amplification to obtain DNA fragments capable of showing polymorphism, which showed different DNA differences after gel electrophoresis. Cluster analysis software was used for analysis, and the results were shown in FIG. 1. As shown in FIG. 1, the RAPD cluster analysis diagram of the *L. rhamnosus* HOM1213 strain is different from that of the commercial *L. rhamnosus* strain, indicating its uniqueness.

Table 1

| Primers | Sequence | SEQ ID NO |
|---|---|---|
| OPA-02 | 5'- TGCCGAGCTG -3' | 2 |
| OPA-18 | 5'- AGGTGACCGT -3' | 3 |
| OPL-07 | 5'- AGGCGGGAAC -3' | 4 |
| OPL-16 | 5'- AGGTTGCAGG-3' | 5 |
| OPM-05 | 5'- GGGAACGTGT -3' | 6 |
| 27F | 5'- AGTTTGATCMTGGCTCAG -3' | 7 |
| 1492R | 5'- GGTTACCTTGTTACGACTT -3' | 8 |

## Example 2. Preparation Process of Viable Bacteria Powder

(1) Culture of the Strain

The strain of *L. rhamnosus* HOM1213 isolated in Example 1 was inoculated into MRS liquid medium at an inoculation amount of 3% of the total amount of the medium, cultured at 37°C for 24 hours, and activated for 2 generations. Inoculated in MRS medium and cultured at 37°C, the viable bacteria count can reach $1 \times 10^{10}$ CFU/mL or more.

(2) Freeze-Drying

The fermented bacterial solution of *L. rhamnosus* HOM1213 strain was centrifuged to collect bacterial sludge. The bacterial sludge was washed with 0.9% sterile physiological saline, mixed with the above-mentioned protective agent (100 g/L skim milk powder), and freeze-dried in a freeze-dryer. The bacterial cake was pulverized by a fine grinder to obtain the freeze-dried bacterial powder, and the viable bacteria count of the freeze-dried bacterial powder was higher than $3.0 \times 10^{11}$ CFU/g.

## Example 3. Gastrointestinal Transit Test

(1) Isolation and Activation of Strains

The commercial strain *L. rhamnosus* GG strain was selected as a control, which was purchased from ATCC (American Type Culture Collection). Each strain (*L. rhamnosus* GG strain and *L. rhamnosus* HOM1213 strain isolated in Example 1) was inoculated into MRS liquid culture medium at an inoculation amount of 3% of the total amount of the medium, cultured at 37°C for 24 h, and activated twice for later use.

(2) Preparation of Artificial Gastric Fluid

Into 16.4 mL of dilute hydrochloric acid, 10 g of pepsin and about 800 mL of water were added, shaken well, and the pH was adjusted to 3.0, the water was added to a constant volume of 1 L, and filtered with a 0.22 $\mu$m microporous membrane for later use.

(3) Preparation of Artificial Intestinal Fluid

6.8 g of potassium dihydrogen phosphate was weighted and dissolved with 500 mL of water, and the pH was adjusted to 6.8 with 0.1 mol/L sodium hydroxide solution. Separately, 10 g of pancreatin and 3 g of oxgall (BD Difco) were weighted, dissolved in an appropriate amount of water. The two solutions were mixed, water was added to 1000 mL, and filtered with a 0.22$\mu$m sterile membrane under sterile environment for later use.

(4) Evaluation of Survival Capacity of Strains in Simulated Artificial Gastrointestinal Fluid

1 mL of the bacterial solution of each strain was taken, centrifuged, the bacteria were collected and 10 mL of the artificial gastric solution was added. The viable bacteria count was counted immediately after uniform mixing, and recorded as $T_0$. Incubated in an incubator at 37°C for 3 h, then the viable bacteria count was counted and recorded as $T_1$. The sample was centrifuged, 10 mL of artificial intestinal fluid was added, mixed well, and incubated in an incubator at 37°C for 3h for viable bacteria count, recorded as $T_2$. Its survival rate was calculated by the following formula:

$$\text{Survival rate in the gastric fluid for 3 hours (\%)} = \frac{CFU\ T_1}{CFU\ T_0} \times 100\%$$

$$\text{Survival rate in the intestinal fluid for 3 hours (\%)} = \frac{CFU\ T_2}{CFU\ T_0} \times 100\%$$

wherein, $T_0$ is the viable bacteria count CFU/mL of the tested strain at 0 h before treatment; $T_1$ is the viable bacteria count of the tested strain after 3 h treatment with artificial gastric fluid; $T_2$ is the viable bacteria count of the test strain after 3 h treatment with artificial gastric fluid and 3 h treatment with artificial intestinal fluid.

It can be seen from Table 2 that the survival rate of the *L. rhamnosus* HOM1213 strain can reach 98% or more, after 3 hours of treatment with simulated artificial gastric fluid; and the survival rate can still reach 80% or more, after 3 hours of treatment with simulated intestinal fluid, indicating that the *L. rhamnosus* HOM1213 strain has a high survival rate in the intestinal tract.

Table 2. Survival Rate of Strains in Simulated Gastrointestinal Fluids

| Strain Name | Survival rate in gastric fluid for 3 hours | Survival rate in intestinal fluid for 3 hours |
|---|---|---|
| HOM1213 Group | 98.79±1.62% | 80.64±4.24% |
| GG Group | 100.84±1.90% | 82.87±2.08% |

**Example 4. Antibiotic Sensitivity**

The drug susceptibility test was performed using the K-B agar method recommended by the National Committee for Clinical Laboratory Standards (NCCLS), the drug sensitivity test, as follows:

(1) The commercial strain *L. rhamnosus* GG strain was selected as the control strain. The *L. rhamnosus* HOM1213 strain and the GG strain were inoculated in MRS liquid medium at an inoculation amount of 3% of the total amount of the medium, cultured at 37°C for 24 hours, and activated continuously for 3 generations for later use.

(2) 1 mL of bacterial solution ($1.5 \times 10^8$ CFU/mL) and 15 mL of MRS solid medium were mixed, and placed in a sterile culture dish, and mixed uniformly. A standard antibiotic susceptibility testing disc was placed after the plate was solidified, and cultured at 37°C for 48 h, and the diameter of the inhibition zone was measured and recorded.

According to the CLSI criteria, the results are shown in Table 3. The *L. rhamnosus* GG strain was used as a commercial strain control, and the results showed that the *L. rhamnosus* HOM1213 strain was resistant to vancomycin, gentamicin, kanamycin and ampicillin, moderately resistant to streptomycin, and sensitive to the remaining four antibiotics. *L. rhamnosus* exhibits natural resistance to vancomycin.

Table 3. Results of susceptibility test of *L. rhamnosus* HOM1213 strain on nine antibiotics.

| Antibiotics | Diameter of Inhibition Zone (mm) | | | HOM1213 Group | GG Group |
|---|---|---|---|---|---|
| | R | | S | | |
| Vancomycin | < 15 | | ≧ 15 | R | R |
| Gentamicin | ≦ 12 | 13-14 | ≧ 15 | R | R |
| Kanamycin | ≦ 13 | 14-17 | ≧ 18 | R | R |
| Ampicillin | ≦ 28 | | ≧ 29 | R | R |
| Clindamycin | ≦ 15 | 16-21 | ≧ 22 | S | S |
| Erythromycin | ≦ 13 | 14-22 | ≧ 23 | S | S |
| Chloramphenicol | ≦ 12 | 13-17 | ≧ 18 | S | S |
| Tetracycline | ≦ 14 | 15-18 | ≧ 19 | S | S |
| Streptomycin | ≦ 11 | 12-14 | ≧ 15 | I | I |

S (susceptible) represents sensitivity; I (intermediate) represents moderate sensitivity; R (resistance) represents drug resistance.

**Example 5. Test for Ability to Inhibit Common Pathogenic Bacteria**

(1) Pathogenic Bacteria Activation

Five pathogenic bacteria: *Escherichia coli (E. coli)* ATCC8739, *Salmonella typhimurium* ATCC14028, *Staphylococcus aureus* ATCC6538, *Pseudomonas aeruginosa* ATCC9027, and *Listeria monocytogenes* ATCC19111 were selected in this experiment. Pathogenic strains were purchased from ATCC (American Type Culture Collection).

The pathogenic strains were inoculated into a nutrient agar medium at an inoculation amount of 3% of the total amount of the medium, and cultured at 37°C and shaking at 200 rpm for 12 h. The indicator bacteria were adjusted to OD = 0.1 with fresh medium for later use.

(2) Strain Activation

In this experiment, the commercial strain *L. rhamnosus* GG strain was selected as the control strain. The commercial strain GG and the *L. rhamnosus* HOM1213 strain isolated in Example 1 were inoculated into an MRS liquid medium at an inoculation amount of 3% of the total amount of the medium, cultured at 37°C for 24 hours, and activated twice to obtain a strain fermentation broth. Centrifuged at 11000 rpm for 10 min, the supernatant was collected for bacteriostatic test, and MRS liquid medium was used as negative control.

(3) Plate Preparation

The sterilized nutrient agar was poured into a petri dish. 100μL of indicator bacteria solution was added to the medium, mixed well, and allowed to stand for coagulation.

(4) Bacteriostasis Experiment

The Oxford cup was gently placed on the plate with sterile forceps, with a certain distance between the holes. 150μL of fermentation supernatant was added to the holes, respectively. After placed in a refrigerator at 4°C for 12 hours, it was cultured in an incubator at 37°C for 18 hours. The diameter of the inhibition zone was observed and measured. The results are shown in Table 4.

Table 4. Inhibitory effect of each group on pathogenic bacteria

| Strain Name | Antibacterial Effect Rating | | | | |
|---|---|---|---|---|---|
| | *E. coli* | *Salmonella* | *Staphylococc us aureus* | *Pseudomonas aeruginosa* | *Listeria monocytogenes* |
| GG Group | ++ | + | + | ++ | + |
| HOM1213 Group | ++ | ++ | + | ++ | ++ |
| Negative Control | - | - | - | - | - |

Note: "-" indicates no antibacterial activity, < 11 mm; "+" indicates 11 mm $\leq$ inhibition zone < 16 mm; "++" indicates 16 mm $\leq$ inhibition zone < 23 mm; and "+++" indicates $\geq$ 23 mm

It can be seen from Table 4 that *L. rhamnosus* HOM1213 strain has inhibitory effects on 5 pathogenic bacteria. Compared with the commercial strain *L. rhamnosus* GG, the *L. rhamnosus* HOM1213 strain exhibits stronger antibacterial ability, which is reflected in the inhibition of *Salmonella* and *Listeria monocytogenes.*

**Example 6. Lactic Acid and Short Chain Fatty Acid Content**

(1) Preparation of Strain Supernatant

The commercial strain *L. rhamnosus* GG strain was selected as the control strain. The *L. rhamnosus* strain HOM1213 isolated in Example 1 and the commercial strain GG were inoculated in MRS liquid medium at an inoculation amount of 3% of the total amount of the medium, cultured at 37°C for 24 hours, and activated continuously for 3 generations. The supernatant was collected for later use after centrifugation at 11,000 rpm and 4°C.

(2) Determination of Lactic Acid, Acetic Acid and Formic Acid Content

4 mL of supernatant was taken, 2 drops of phenolphthalein were added, the color was adjusted with KOH solution until a slight color change occurred, and diluted to 5 mL with deionized water. Centrifuged at 11,000 rpm at 4°C for 10 minutes, and the sample was set aside for later use.. Different concentrations of lactic acid, acetic acid, formic acid and samples were taken respectively in different 10 mL headspace injection vials, then 2 mL of phosphate buffer solution, 2 mL of derivatization reagent and 1 mL of acetone were added. The vials were sealed by capping, and reacted in a water bath at 100°C for 40 min. After cooling to room temperature, the derivatized samples were transferred in a 2 mL centrifuge tube, and an equal volume of isooctane vortex extraction was added for vortex extraction, and an isooctane layer was taken for gas chromatography (GC) analysis. The results are shown in Table 5.

Table 5. Determination of Lactic Acid, Acetic Acid, and Formic Acid Content ( x $\pm$ SD)

| | Lactic Acid ($\mu$g/mL) | Acetic Acid ($\mu$g/mL) | Formic Acid ($\mu$g/mL) |
|---|---|---|---|
| MRS Control Group | 377.67$\pm$0.47 | 1949.42$\pm$10.04 | 98.69$\pm$14.02 |
| HOM1213 Group | 15305.57$\pm$182.94** | 2305.54$\pm$90.45** | 87.40$\pm$9.17 |
| GG Group | 11765.35$\pm$122.85** | 2048.82$\pm$26.11** | 100.02$\pm$6.18 |

**: $p < 0.01$ compared with MRS control group

It can be seen from Table 5 that both *L. rhamnosus* strains HOM1213 and GG produced a high content of lactic acid and a certain content of acetic acid. Lactic acid and acetic acid can inhibit the proliferation of pathogenic bacteria and help maintain a healthy intestinal flora.

**Example 7. In Vitro Cytokine Secretion Promoting Test**

The commercial strain *L. rhamnosus* GG was selected as the control strain. The *L. rhamnosus* strain HOM1213 isolated in Example 1 and the commercial strain GG were inoculated in MRS liquid medium at an inoculation amount of 3% of the total amount of the medium, fermented and cultured at 37°C for 24 h, and activated continuously for 3 generations.

After centrifugation at 11000 rpm for 10 min, the bacteria were collected and adjusted to the working concentration (about $5.0 \times 10^5$ CFU/mL) using antibiotic-free DMEM complete medium. Mouse macrophages RAW264.7 (about $5 \times 10^5$ cells/mL) were added to a 24-well culture plate with 1 mL per well. After 2h of adhesion, the medium was discarded, and 1 mL of bacteria-containing medium was added to each well. A blank control group was set, into which 1 mL of DMEM medium was added. The cell supernatant was collected after co-culture for 24 h.

The contents of TNF-$\alpha$ and IL-6 in the cell supernatant were determined by enzyme-linked immunosorbent assay (ELISA) according to the instructions of the kit.

Table 6. *L. rhamnosus* HOM1213 strain promotes the secretion of cytokine by mouse macrophages ( x $\pm$ SD)

| Group | TNF-$\alpha$ secretion (pg/mL) | IL-6 secretion (pg/mL) |
|---|---|---|
| Blank Control Group | 59$\pm$12 | 0$\pm$0 |
| HOM1213 Group | 6294$\pm$1433* | 35$\pm$1** |
| GG Group | 5678$\pm$1829* | 59$\pm$3** |

Note: *: p < 0.05 compared with the blank control group, **: p < 0.01 compared with the blank control group

The results were shown in Table 6. *L. rhamnosus* HOM1213 strain can significantly increase the secretion of TNF-$\alpha$ and IL-6 by RAW264.7 cells. It can be seen that the *L. rhamnosus* HOM1213 strain has potential immunomodulatory ability.

## Example 8. Exploration of In Vitro Blood Glucose Lowering Efficacy and Mechanism

(1) Modified MRS Liquid Medium

Into the MRS medium (OXOID), 0.05% of L-cysteine hydrochloride was added on the basis of the finished product medium, stirred evenly, and sterilized at 121°C for 20 min for later use.

(2) Strain Activation and Sample Treatment

The commercial strain *L. rhamnosus* GG was selected as a positive control, and the strain *L. rhamnosus* HOM1213 isolated in Example 1 and the commercial strain GG were inoculated into a fresh modified MRS liquid medium at an inoculation amount of 1% of the total amount of the medium respectively, allowed to stand at 37°C for anaerobic culture for 24 h, and activated for 3 generations. The bacterial solution was centrifuged at 11000 rpm for 10 min, and washed three times with phosphate buffer solution (PBS). The viable bacteria count was adjusted to $4 \times 10^{10}$ CFU/mL, anaerobic culture was performed for 8 h, centrifugation was performed at 11000 rpm for 10 min, and the supernatant was collected for later use.

(3) Determination of DPP-4 Inhibitory and $\alpha$-Glucosidase Inhibitory

In vitro screening of blood glucose-lowering function was performed using a DPP-4 inhibitor screening kit (Abnova # KA1311) and an $\alpha$-glucosidase inhibitor screening kit (Biovision # K938). The inhibition rates of each sample on the above two enzymes were calculated, and the results were shown in Table 7 and FIG. 2.

Table 7 shows the inhibition rate of each group on DPP-4 and the inhibition rate of $\alpha$-glucosidase (dose x $\pm$ SD).

| Strains | DPP-4 inhibition rate (%) | p value | $\alpha$-Glucosidase Inhibition Rate (%) | p value |
|---|---|---|---|---|
| HOM1213 Group | 17.21% $\pm$ 3.46% | - | 16.79% $\pm$0.62% | - |
| GG Group | 12.24% $\pm$ 1.77% | 0.212 | 6.45% $\pm$ 0.71% | 0.004** |

**: Significant difference compared with *L. rhamnosus* HOM1213 strain group (p < 0.01)

It can be seen from Table 7 and FIG. 2 that the *L. rhamnosus* HOM1213 strain has a high inhibition rate on DPP-4 and $\alpha$-glucosidase. In the above two indicators, the strain group of *L. rhamnosus* HOM1213 was higher than the strain group of *L. rhamnosus* GG. In summary, the *L. rhamnosus* HOM1213 strain has the potential to regulate blood glucose, and may be derived from the production of higher levels of DPP-4 and $\alpha$-glucosidase inhibitors.

## Example 9. Stimulation of NCI-H716 Cells to Secrete GLP-1 by *L. rhamnosus* HOM1213 Strain

(1) Culture of NCI-H716 Cells

In this experiment, NCI-H716 cells (purchased from Cell Bank of the Chinese Academy of Sciences) were selected. NCI-H716 cells were suspended and grown in RPMI-1640 (Gibco) medium containing 10% fetal bovine serum (Hyclone) and 1% double antibiotics (penicillin and streptomycin, Hyclone) in an incubator at 37°C, 5% $CO_2$.

(2) Strain Activation and Sample Treatment

The commercial strain *L. rhamnosus* GG strain was selected as a positive control. The *L. rhamnosus* HOM1213 strain isolated in Example 1 and the commercial strain GG were inoculated in MRS liquid medium at an inoculation amount of 3% of the total amount of the medium, cultured statically at 37°C for 24 h, and activated for 3 generations. Each bacterial solution was centrifuged at 11000 rpm for 10 min and washed three times with Krebs buffer (Sigma). The viable bacteria count was adjusted to $1 \times 10^{10}$ CFU/mL for later use.

(3) GLP-1 Endocrine Assay

NCI-H716 cells were inoculated in a 24-well plate (Corning) coated with matrigel at a density of $1.5 \times 10^6$ cells/well. Endocrine differentiation medium was added, and the cells were cultured in an incubator at 37°C and 5% $CO_2$ for 2 days for endocrine differentiation assay. The endocrine differentiation medium was a high-glucose DMEM (Gibco) medium containing 10% fetal bovine serum and 1% double antibiotics.

After 2 days, the DMEM medium was replaced with Krebs-Ringer buffer, and the buffer contained $1 \times 10^{10}$ CFU/mL of probiotic strains, respectively. After culturing for 2 h, centrifuged at 8000 rpm for 10 min, and the supernatant was collected. 50µg/mL of phenylmethylsulfonyl fluoride (Roche) and 10µg/mL of sitagliptin (Sigma) were added to the supernatant, and then the concentration of GLP-1 was detected using an ELISA kit (Raybiotech). The results are shown in Table 8.

Table 8. GLP-1 secretion yield in NCI-H716 cells stimulated by different strains ( x ±SD)

| Strains | GLP-1(pg/mL) | p value |
|---|---|---|
| HOM1213 Group | 1781.83±22.22 | |
| GG Group | 962.34±44.62** | 0.000 |

**: Significant difference compared with *L. rhamnosus* HOM1213 strain group (p < 0.01)

It can be seen from Table 8 that the *L. rhamnosus* HOM1213 strain can stimulate NCI-H716 cells to secrete a high content of GLP-1 at a concentration of 1781.83 ± 22.22 pg/mL. Compared with *L. rhamnosus* commercial strain GG, *L. rhamnosus* HOM1213 strain has a very significant difference (p < 0.01). The results show that *L. rhamnosus* HOM1213 strain has the potential to lower blood glucose, and the mechanism may be to stimulate intestinal L cells to produce high levels of GLP-1.

GLP-1 is an incretin, which promotes insulin secretion by pancreatic β cells and inhibits glucagon secretion by α cells in a glucose concentration-dependent manner, has a good effect of lowering postprandial blood glucose. When glucose concentration is lower than normal, it does not stimulate insulin secretion, thereby preventing hypoglycemia.

**Example 10. In Vitro Antioxidant Capacity Test**

(1) Strain Activation

The *L. rhamnosus* HOM1213 isolated in Example 1 and GG strains were inoculated in an MRS liquid medium at an inoculation amount of 3% of the total amount of the medium, fermented and cultured at 37°C for 24 h, activated continuously for 3 generations. It was centrifuged at 11000 rpm for 10 min, and the bacterial cells were washed with PBS buffer, resuspended and centrifuged, repeated three times. The count of bacteria was adjusted to $2 \times 10^{10}$ CFU/ml for later use.

(2) Detection of Antioxidant Indicators

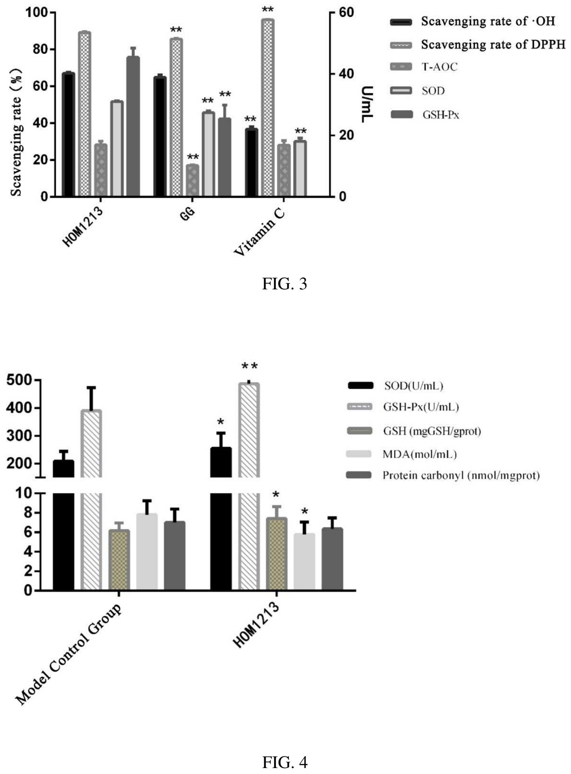

Indicators included total antioxidant capacity (T-AOC), superoxide dismutase (SOD), glutathione peroxidase (GSH-Px), glutathione (GSH), hydroxyl radical scavenging test (·OH), and DPPH radical scavenging test. The DPPH free radical scavenging test used a colorimetric method, and its principle was that the free radical scavenger donated an electron to pair with the lone pair electron of the DPPH free radicals, so that its own color changed from purple to yellow. Its absorbance at the wavelength of 517 nm decreased, and the degree of change has a linear relation to the degree of free radical scavenging, that is, the stronger the scavenging ability of the free radical scavenger, the lower the absorbance. Other indicators were determined by using a kit purchased from Nanjing Jiancheng Company, according to operation instructions. 0.05% vitamin C was used as a positive control to determine various antioxidant indicators of *L. rhamnosus* HOM1213 and GG strains suspensions. The results were shown in Table 9 and FIG. 3.

Table 9. Detection of in vitro antioxidant capacity ( x $\pm$ SD)

| Strain Name | T-AOC (U/mL) | SOD (U/mL) | GSH-PX (U/mL) | GSH (mgGSH/L ) | ·OH Scavenging Rate (%) | DPPH Scavenging Rate (%) |
|---|---|---|---|---|---|---|
| HOM1213 | 16.94±1.17 | 31.02±0.28 | 45.41±3.02 | 2.06±0.31 | 67.04±0.63% | 89.09±0.57% |
| GG | 10.24±0.25 ** | 27.42±0.56 ** | 25.36±4.53 ** | 1.07±0.03 ** | 64.84±1.36% | 85.61±0.34% ** |
| Vitamin C | 16.90±1.42 | 18.07±1.08 ** | ND | 0.33±0.06 ** | 36.70±1.33% ** | 96.14±0.08% ** |

Note: ND: not detected; **: very significant difference compared with HOM1213 group ($p < 0.01$)

It can be seen from Table 9 and FIG. 3 that the *L. rhamnosus* HOM1213 strain has high T-AOC, SOD, and GSH-Px activities, and has high hydroxyl radical and DPPH radical scavenging rates, especially showing outstanding performance in SOD enzyme activity, hydroxyl radical scavenging, and DPPH radical scavenging. This indicated that the *L. rhamnosus* HOM1213 strain has excellent antioxidant activity.

**Example 11. Improving Antioxidant Levels and Delays Aging in Mice**

(1) Mouse Sources

30 healthy SPF-grade female mice (22-26g) bred in Kunming by Beijing Huafukang Biotechnology Co., Ltd were selected. The experimental animals were housed in the SPF-grade animal chamber of the Health Food Functional Testing Center, School of Applied Arts and Sciences, Beijing Union University. Maintenance feed was produced by Beijing Huafukang Biotechnology Co., Ltd.

(2) D-Galactose Oxidation Damage Model

30 mice were divided into 3 groups of 10 mice each. Two groups of mice were induced to develop the model by subcutaneous injection of D-galactose prepared with physiological saline at a dose of 300 mg/kg BW into the back of the neck. The injection amount was 0.1 mL/10 g, once a day, for 6 consecutive weeks. The blood samples were taken to measure malondialdehyde levels. Two groups were randomly divided according to the level of malondialdehyde, including the model control group and the HOM1213 group. The other group was a blank control group, which received daily subcutaneous injections of an equal volume of physiological saline into the back of the neck during the modeling period.

(3) Dosage Design

After successful modeling, the sample was orally administered once a day, with the gavage volume of 10 mL/kg BW. The *L. rhamnosus* HOM1213 strain group was administered $1 \times 10^9$ CFU bacteria solution, while the blank control group and the model control group were replaced with sterile water, and the daily gavage volume was the same as that of the sample group. Each group was administered maintenance feed. After continuous gavage for 45 days, various indicators in mice were measured. While the samples were administered, the *L. rhamnosus* HOM1213 strain group and the model control group were continuously injected with the same dose of D-galactose, and the blank control group was injected with the same amount of physiological saline.

The specific experimental method is based on "Technical Specification for Inspection and Evaluation of Health Foods" (2012 edition), and the experimental result data was statistically analyzed by SPSS software.

(4) Indicator Determination

Kits required in the test were all purchased from Nanjing Jiancheng Bioengineering Research Institute.

A. Determination of Lipid Peroxidation - Malondialdehyde (MDA) Content in Serum

0.5 mL of blood was collected from the venous plexus of angular vein of eyes in mice, centrifuged at 3000 r/min for 10 min, and the content of malondialdehyde in serum was determined by using a kit. The results were shown in Table 12.

B. Determination of the Protein Carbonyl Content of Protein Oxidation Products in Liver Tissue

A certain amount of liver tissue was taken and rinsed in ice-cold physiological saline to remove the blood stains on the surface, and then wiped dry with the filter paper. Protein carbonyl content was determined using a protein carbonyl kit (A087-1-2, Nanjing Jiancheng Bioengineering). Specific method: Reagent I was added at a weight-to-volume ratio of 1:9, and mechanically homogenized in an ice bath. The homogenate was centrifuged at 3000 r/min for 10 min with a centrifuge, and the supernatant (10% liver tissue homogenate) was used for determination. 450$\mu$L of 10% liver tissue homogenate was taken, 50$\mu$L of reagent II was added, placed at room temperature for 10 min, then centrifuged at a rotation speed of 11000 r/min for 10 min, and the supernatant was taken to be tested. The results are shown in Table 13.

C. Antioxidant Enzymes

0.5 mL of blood was collected from the venous plexus of angular vein of elderly mice eye, centrifuged at 3000 r/min for 10 min. The activity of superoxide dismutase (SOD) in serum was measured using a superoxide dismutase activity kit. The results are shown in Table 14.
10$\mu$L of blood was collected from the venous plexus of angular vein of elderly mice eye, and distilled water was added to a final volume of 1 mL, to prepare 1:99 hemolysate. The activity of glutathione peroxidase (GSH-Px) was measured using the glutathione peroxidase kit. The results are shown in Table 15.

D. Determination of Antioxidant - Reduced Glutathione (GSH) Content

A certain amount of liver was cut, rinsed with physiological saline, wiped dry, weighed, and minced. It was placed in a glass homogenizer and homogenized with cold physiological saline to prepare a 10% tissue homogenate. The homogenate was centrifuged at 2500 r/min for 10 min, the supernatant was taken, and the GSH kit was used to determine the GSH content. The results are shown in Table 16.

Table 10. Serum malondialdehyde (MDA) content in mice after modeling ( x $\pm$ SD)

| Group | Number of animals (n) | MDA (nmol/mL) | p value |
|---|---|---|---|
| Blank Control Group | 10 | 6.31$\pm$0.85** | 0.004 |
| Model Control Group | 10 | 7.77$\pm$1.11 | - |
| HOM1213 Group | 10 | 7.89$\pm$1.14 | 0.819 |

**: Comparison between the model control group and the blank control group, p < 0.01

It can be seen from Table 10 that after 6 weeks of D-galactose modeling in mice via subcutaneous injection, the model group was randomly divided into a model control group and a sample group HOM1213 group according to the serum malondialdehyde content of the mice. There was no significant difference (p > 0.05) between the HOM1213 group and the model control group, indicating that the serum malondialdehyde content of the mice was relatively balanced among the model groups. Compared with the blank control group, the model control group showed a significant increase (p < 0.01) in the serum malondialdehyde content, indicating that the D-galactose oxidative damage model was successfully established.

Table 11. Changes in body weight of the mice before and after sample administration ( x $\pm$ SD)

| Group | Number of Animals | before sample administration | | After sample administration | |
|---|---|---|---|---|---|
| | | Body Weight (g) | p value | Body Weight (g) | p value |
| Model Control Group | 10 | 33.3$\pm$2.1 | - | 36.4$\pm$2.7 | - |
| HOM1213 Group | 10 | 32.8$\pm$4.1 | 0.717 | 36.0$\pm$3.1 | 0.731 |
| Blank Control Group | 10 | 34.0$\pm$3.4 | 0.626 | 38.4$\pm$2.9 | 0.152 |

It can be seen from Table 11 that there were no significant differences (p > 0.05) in body weight of the mice between the model control group and the blank control group before and 45 days after oral administration of the sample, and no significant differences (p > 0.05) between the sample group and the model control group. That is, the sample had no adverse effect on the body weight of the mice.

Table 12. Effect of *L. rhamnosus* HOM1213 strain on serum malondialdehyde in mice ( x ± SD)

| Group | Number of animals (n) | MDA (nmol/mL) | p value |
|---|---|---|---|
| Model Control Group | 10 | 7.81±1.43 | - |
| HOM1213 Group | 10 | 5.78±1.27** | 0.003 |
| **: Comparison between the sample group and the model control group, p < 0.01 | | | |

It can be seen from Table 12 that after oral administration of sample to mice for 45 days, compared with the model control group, the serum malondialdehyde content in the *L. rhamnosus* HOM1213 strain group was reduced, and there was a very significant difference (p < 0.01). This indicates that the sample of *L. rhamnosus* HOM1213 strain group can significantly reduce the content of malondialdehyde in the serum of D-galactose oxidative damage model mice. Malondialdehyde is the major product of polyunsaturated fatty acid peroxidation. Malondialdehyde content has been widely used to determine the level of oxidative damage in vivo.

Table 13. Effect of *L. rhamnosus* HOM1213 strain group on protein carbonyl content in liver tissue of the mice ( x ± SD)

| Group | Number of animals (n) | Protein carbonyl (nmol/mgprot) | p value |
|---|---|---|---|
| Model Control Group | 10 | 7.02±1.36 | - |
| HOM1213 Group | 10 | 6.36±1.12 | 0.256 |

Protein carbonylation is a type of protein oxidative damage, which refers to the conversion of amino acid residue side chains into carbonyl products after being attacked by oxygen radicals. Carbonyl modification causes changes in protein structure, leading to cell and tissue lesions, and participating in body aging and apoptosis. It can be seen from Table 13 that after oral administration of sample to mice for 45d, compared with the model control group, the protein carbonyl content of liver tissue in the L. *rhamnosus* HOM1213 strain group was reduced, but the differences were not significant (p > 0.05).

Table 14. Effect of *L. rhamnosus* HOM1213 strain group on superoxide dismutase (SOD) activity in serum of the mice ( x ± SD)

| Group | Number of animals (n) | SOD activity (U/mL) | p value |
|---|---|---|---|
| Model Control Group | 10 | 209±35 | |
| HOM1213 Group | 10 | 255±55* | 0.037 |
| *: Comparison between the sample group and the model control group, p < 0.05 | | | |

It can be seen from Table 14 that after oral administration of the sample to mice for 45d, compared with the model control group, the SOD activity in serum in the *L. rhamnosus* HOM1213 strain group was improved, and there was a significant difference (p < 0.05). The *L. rhamnosus* HOM1213 strain can increase the SOD level of the body to resist oxidative stress levels.

Table 15. Effect of *L. rhamnosus* HOM1213 strain group on the glutathione peroxidase activity in whole blood of the mice ( x ± SD)

| Group | Number of animals (n) | GSH-Px Activity (U) | p value |
|---|---|---|---|
| Model Control Group | 10 | 390±83 | |
| HOM1213 Group | 10 | 487±57** | 0.007 |
| **: Comparison between the sample group and the model control group,p < 0.01 | | | |

It can be seen from Table 15 that after oral administration of the sample to mice for 45d, the glutathione peroxidase activity of whole blood in the *L. rhamnosus* HOM1213 strain group increased, with a very significant difference (p < 0.01), that is, the *L. rhamnosus* HOM1213 strain group can improve the whole blood glutathione peroxidase activity of the D-galactose oxidative damage model mice to resist the oxidative stress level.

Table 16. Effects of the strain group of *L. rhamnosus* HOM1213 strain on reduced glutathione content in liver tissue of mice ( x $\pm$ SD)

| Group | Number of animals (n) | GSH (mgGSH/gprot) | p value |
|---|---|---|---|
| Model Control Group | 10 | 6.17$\pm$0.79 | |
| HOM1213 Group | 10 | 7.41$\pm$1.23* | 0.015 |

*: Comparison between the sample group and the model control group, p < 0.05

It can be seen from Table 16 that after oral administration of the sample to mice for 45d, compared with the model control group, the content of reduced glutathione in the liver tissue of the *L. rhamnosus* HOM1213 strain group was increased, and there was a significant difference (p < 0.05). That is, *L. rhamnosus* HOM1213 strain can increase the content of reduced glutathione in liver tissue of mice with D-galactose oxidation damage model.

According to the determination method of "Methods for Testing and Evaluation of Functional Health Foods" (2022 edition) for antioxidant animal experiments, if three of the four indicators of lipid oxidation products, protein oxidation products, antioxidant enzymes and antioxidant substances are positive, the test sample can be considered to contribute to the positive result in the antioxidant animal experiment. This indicates that the *L. rhamnosus* HOM1213 strain has antioxidation and anti-aging functions.

FIG. 4 shows the effect of the *L. rhamnosus* HOM1213 strain of the present invention on antioxidant and anti-aging indicators in vivo of the oxidative damage model mice. After oral administration of the sample to mice for 45d, compared with the model control group, the serum superoxide dismutase (SOD) activity in the *L. rhamnosus* strain HOM1213 group was increased, with a significant difference (p < 0.05). The whole blood glutathione peroxidase (GSH-Px) activity was increased, with a very significant difference (p < 0.01). The reduced glutathione (GSH) content in liver tissue was also increased, with a significant difference (p < 0.05). The serum malondialdehyde (MDA) content was reduced, with a very significant difference (p < 0.01). The liver tissue protein carbonyl content was reduced, but the difference was not significant (p > 0.05). Therefore, the HOM1213 group significantly increased serum SOD and GSH-Px activities and GSH levels, and significantly decreased MDA content in mice model with D-galactose oxidative damage.

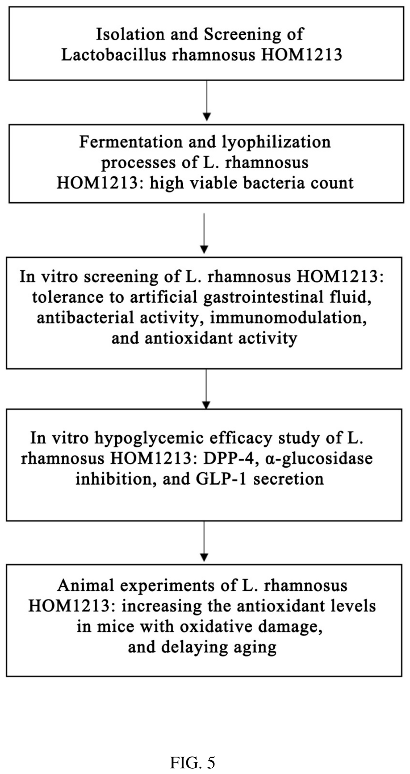

The entire process of the present invention is shown in the process flow diagram of FIG. 5.

**Example 12. Fermented Bovine Milk Test**

12g of skim milk powder, 2 g of glucose, and 2 g of yeast powder were weighed, double-distilled water was supplemented to a final volume of 100 mL, stirred uniformly, homogenized in a high-pressure homogenizer (60°C, 22 MPa), sterilized at 95°C for 10 min, and cooled to 37°C for later use.

The seed solution of the *L. rhamnosus* HOM1213 strain isolated in Example 1 was inoculated into an MRS liquid medium, cultured at 37°C for 18 h. It was subcultured twice according to this method, to obtain a highly viable bacterial solution for later use.

The bacterial solution was eluted and resuspended with 0.9% physiological saline, inoculated into bovine milk at an inoculation amount of $1 \times 10^7$ CFU/mL, mixed uniformly, and allowed to stand at a constant temperature of 37°C for fermentation for 16h to obtain a fermented dairy product. The content of active *L. rhamnosus* in the fermented milk is higher than $6.0 \times 10^8$ CFU/mL, the pH can reach 4.50. The fermented milk has a rich and mellow flavor and a sweet and sour taste.

**Example 13. Fermented Oat Milk Test**

120g of enzymolyzed oat powder and 20 g of yeast powder were weighed, and double-distilled water was supplemented to a final volume of 1000 mL, homogenized at 200 bar, sterilized at 90°C for 10 min, and cooled to 37°C for later use.

The seed solution of the *L. rhamnosus* HOM1213 strain isolated in Example 1 was inoculated into an MRS liquid medium, cultured at 37°C for 18 h. Subculture twice to obtain a high-concentration bacterial solution, and preserved at low temperature for later use.

The bacterial solution was eluted and resuspended with 0.9% physiological saline, inoculated into oat milk at an inoculation amount of $1 \times 10^7$ CFU/mL, mixed well, and allowed to stand at a constant temperature of 37°C for fermentation for 6h to obtain fermented oat milk. The viable bacteria count of *L. rhamnosus* in the fermented milk is higher than $5.0 \times 10^8$ CFU/mL, the pH can be reduced to 4.35. The fermented milk has rich flavor.

The above description is only preferred embodiments of the present disclosure, and it should be noted that for those skilled in the art, several improvements and modifications can be made without departing from the principle of the present disclosure, and these improvements and modifications should also be considered within the protection scope of the present invention.

**Claims**

1. A *Lactobacillus rhamnosus (L. rhamnosus)* strain, wherein the deposit number of the *L. rhamnosus* strain is CGMCC No. 25682.

2. The *L. rhamnosus* strain according to claim 1, wherein the *L. rhamnosus* strain comprises a 16S rRNA gene sequence represented by SEQ ID NO: 1.

3. A fermentation product obtained by fermentation using the *L. rhamnosus* strain according to claim 1 or 2.

4. The fermentation product according to claim 3, wherein the fermentation product is a fermented bovine milk product; optionally, the content of viable *L. rhamnosus* strain in the fermented bovine milk product is $6.0 \times 10^8$ CFU/mL or more.

5. The fermentation product according to claim 3, wherein the fermentation product is a fermented oat milk product; optionally, the count of viable *L. rhamnosus* strain in the fermented oat milk product is $5.0 \times 10^8$ CFU/mL or more.

6. A viable bacteria preparation comprising the *L. rhamnosus* strain according to claim 1.

7. The viable bacteria preparation according to claim 6, wherein the viable bacteria preparation comprises $3.0 \times 10^{11}$ CFU/g or more of the viable bacteria.

8. A food or health care product comprising the viable bacteria preparation according to claim 6.

9. The food or health care product according to claim 8, wherein the food is selected from one or more of a group consisting of milk powder, solid beverage, fermented dairy product, dairy-containing beverage and cheese.

10. The food or health care product according to claim 9, wherein the fermented dairy product is selected from a group consisting of fermented bovine milk product and fermented oat milk product.

11. Use of the *L. rhamnosus* strain according to claim 1 in the preparation of a medicament for scavenging free radicals, responding to oxidative stress state, antioxidation and anti-aging.

12. The use according to claim 11, wherein the *L. rhamnosus* strain is used to increase the levels of superoxide dismutase, glutathione peroxidase and glutathione, and reduce the level of malondialdehyde.

13. The use according to claim 12, wherein the *L. rhamnosus* strain is used to scavenge excess free radicals, and increase the scavenging rate of hydroxyl radicals and DPPH radicals.

14. Use of the *L. rhamnosus* strain according to claim 1 in the preparation of a medicament for producing DPP-4 and $\alpha$-glucosidase inhibitors and stimulating NCI-H716 cells to produce GLP-1.

15. The use according to claim 14, wherein the *L. rhamnosus* strain is used to assist in lowering blood glucose.

16. Use of the *L. rhamnosus* strain according to claim 1 in the preparation of a medicament for modulating immunity.

17. The use according to claim 16, wherein the *L. rhamnosus* strain is used to increase the level of cytokines TNF-$\alpha$ and/or IL-6.

18. Use of the *L. rhamnosus* strain according to claim 1 for increasing the content of lactic acid or short-chain fatty acid.

19. The use according to claim 18, wherein the short-chain fatty acid is acetic acid.

20. The use according to claim 18, wherein the *L. rhamnosus* strain is used to maintain the balance of intestinal flora.

21. A method for preparing the viable bacteria preparation according to claim 6 or 7, comprising the following steps:

propagation - culturing the *L. rhamnosus* strain according to claim 6 or 7 in an optimized liquid medium; collecting bacteria;
adding a protective agent for resuspension, freeze-drying under vacuum, and pulverizing to obtain the viable bacteria preparation.

FIG. 1

FIG. 2

FIG. 3

FIG. 4

Isolation and Screening of
Lactobacillus rhamnosus HOM1213

Fermentation and lyophilization
processes of L. rhamnosus
HOM1213: high viable bacteria count

In vitro screening of L. rhamnosus HOM1213:
tolerance to artificial gastrointestinal fluid,
antibacterial activity, immunomodulation,
and antioxidant activity

In vitro hypoglycemic efficacy study of L.
rhamnosus HOM1213: DPP-4, α-glucosidase
inhibition, and GLP-1 secretion

Animal experiments of L. rhamnosus
HOM1213: increasing the antioxidant levels
in mice with oxidative damage,
and delaying aging

FIG. 5

## INTERNATIONAL SEARCH REPORT

| International application No. |
|---|
| **PCT/CN2024/100580** |

| A. | CLASSIFICATION OF SUBJECT MATTER |
|---|---|

C12N1/20(2006.01)i; A23L33/135(2016.01)i; A61K35/747(2015.01)i; A61P3/10(2006.01)i; A61P39/06(2006.01)i; A61P37/02(2006.01)i; C12R1/225(2006.01)i; C12P7/56(2006.01)i

According to International Patent Classification (IPC) or to both national classification and IPC

| B. | FIELDS SEARCHED |
|---|---|

Minimum documentation searched (classification system followed by classification symbols)

IPC: C12N, A23L, A61K, A61P, C12R, C12P

Documentation searched other than minimum documentation to the extent that such documents are included in the fields searched

Electronic data base consulted during the international search (name of data base and, where practicable, search terms used)

VEN, CNABS, PubMed, DWPI, CNTXT, WOTXT, USTXT, EPTXT, JPTXT, CNKI, Web of Science, 百度学术, BAIDU SCHOLAR, Patentics, 中国生物序列检索系统, China Biological Sequence Search System, NCBI Genbank, EBI, STNext: 申请人, 发明人, 序列1, 鼠李糖乳杆菌, Lactobacillus rhamnosus, CGMCC No.25682, HOM1213, antioxidant, 抗氧化

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 117323349 A (KELI CO., LTD.) 02 January 2024 (2024-01-02) claims 1-11 | 1-21 |
| PX | CN 117327604 A (KELI CO., LTD.) 02 January 2024 (2024-01-02) claims 1-13 | 1-21 |
| PX | CN 117327608 A (KELI CO., LTD.) 02 January 2024 (2024-01-02) claims 1-10 | 1-21 |
| A | CN 114774315 A (WECARE PROBIOTICS (SUZHOU) CO., LTD.) 22 July 2022 (2022-07-22) abstract, and claims 1-10 | 1-21 |
| A | CN 114874951 A (INNER MONGOLIA YIKANG HEALTH DEVELOPMENT CO., LTD.) 09 August 2022 (2022-08-09) abstract | 1-21 |
| A | US 2016113975 A1 (JIANGNAN UNIVERSITY) 28 April 2016 (2016-04-28) abstract | 1-21 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **29 September 2024** | **10 October 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/100580** |

### C. DOCUMENTS CONSIDERED TO BE RELEVANT

| Category* | Citation of document, with indication, where appropriate, of the relevant passages | Relevant to claim No. |
| --- | --- | --- |
| A | 郑柳青等 (ZHENG, Liuqing et al.). "鼠李糖乳杆菌LR-ZB1107-01的安全性评价及其益生特性初步研究 (Assessment of Safety and Probiotic Characteristics of Lactobacillus rhamnosus ZB1107-01)" 食品工业科技 (Science and Technology of Food Industry), Vol. 41, No. 11, 31 December 2020 (2020-12-31), pages 111-116 | 1-21 |
| A | Fang, S. "Lacticaseibacillus Rhamnosus Strain 13835 16S Ribosomal RNA Gene, Partial Sequence" GenBank: MW450433.1, 13 January 2021 (2021-01-13), sequence part | 1-21 |

Form PCT/ISA/210 (second sheet) (July 2022)

## INTERNATIONAL SEARCH REPORT

International application No.

**PCT/CN2024/100580**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

| International application No. |
| --- |
| **PCT/CN2024/100580** |

| Patent document cited in search report | | | Publication date (day/month/year) | Patent family member(s) | Publication date (day/month/year) |
| --- | --- | --- | --- | --- | --- |
| CN | 117323349 | A | 02 January 2024 | None | |
| CN | 117327604 | A | 02 January 2024 | None | |
| CN | 117327608 | A | 02 January 2024 | None | |
| CN | 114774315 | A | 22 July 2022 | None | |
| CN | 114874951 | A | 09 August 2022 | None | |
| US | 2016113975 | A1 | 28 April 2016 | US 9649347 B2 | 16 May 2017 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310744495
- CN 202310745739
- CN 202310744266

**Non-patent literature cited in the description**

- Technical Specification for Inspection and Evaluation of Health Foods. 2012
- Methods for Testing and Evaluation of Functional Health Foods. 2022